(19) Europäisches Patentamt
European Patent Office
Office européen des brevets

(11) **EP 3 325 040 B1**

(12) **EUROPEAN PATENT SPECIFICATION**

(45) Date of publication and mention
of the grant of the patent:
**06.11.2024 Bulletin 2024/45**

(21) Application number: **16745385.1**

(22) Date of filing: **22.07.2016**

(51) International Patent Classification (IPC):
***A61M 1/36*** *(2006.01)*

(52) Cooperative Patent Classification (CPC):
**A61M 1/3681;** A61M 1/0209; A61M 1/3683

(86) International application number:
**PCT/US2016/043759**

(87) International publication number:
**WO 2017/015639 (26.01.2017 Gazette 2017/04)**

(54) **FLOW-THROUGH PATHOGEN REDUCTION**

VERRINGERUNG VON DURCHFLUSSPATHOGENEN

RÉDUCTION D'AGENTS PATHOGÈNES DANS UN FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.07.2015 US 201562195995 P**

(43) Date of publication of application:
**30.05.2018 Bulletin 2018/22**

(73) Proprietor: **Terumo BCT Biotechnologies, LLC
Lakewood CO 80215 (US)**

(72) Inventors:
• **DODD, Jon A.
Littleton, Colorado 80127 (US)**
• **CLEMENT, Daniel Russell
Golden, Colorado 80401 (US)**

(74) Representative: **J A Kemp LLP
80 Turnmill Street
London EC1M 5QU (GB)**

(56) References cited:
WO-A1-2009/079383     US-A- 2 254 994
US-A1- 2002 096 648     US-A1- 2003 146 162

## Description

### Background

There is a constant need for ensuring that fluids, such as biological fluids, can be safely administered to patients without the fear of infection. For example, blood and blood components that are infused into a patient may contain pathogens that when infused into a patient may infect the patient. A need therefore exists to be able to reduce pathogens in a fluid efficiently and effectively. WO 2009/079383 A1 discloses systems, methods and devices for blood treatment.

Embodiments have been made in light of these and other considerations. However, the relatively specific problems discussed above do not limit the applicability of the embodiments.

### Summary

The summary is provided to introduce aspects of some embodiments in a simplified form, and is not intended to identify key or essential elements, nor is it intended to limit the embodiments. The invention is defined in the appended claims.

Embodiments provide for reducing pathogens in a fluid. The embodiments may include systems, and apparatuses. One embodiment provides for a flow cell with a plurality of channels through which a fluid to be pathogen reduced is flowed. The flow cell, and fluid, may be illuminated from more than one direction to pathogen reduce the fluid. The fluid may include a photosensitizer to aid in the pathogen reduction process.

Other embodiments provide for a system that reduces pathogens in a fluid. The system may include a flow cell with a plurality of channels and an illumination system. Each of the plurality of channels has a depth that is less than 0.150 mm. The illumination system may be configured to illuminate each of the plurality of channels from a plurality of sides.

### Brief Description of the Drawings

Non-limiting and non-exhaustive embodiments are described with reference to the following figures.

FIG. 1A illustrates a flow-through pathogen reduction system according to one embodiment.
FIG. 1B illustrates a flow-through pathogen reduction system according to another embodiment.
FIG. 2 illustrates a perspective view of a flow cell according to an embodiment.
FIG. 3 illustrates a top view of the flow cell shown in FIG. 2.
FIG. 4 illustrates a cross-sectional view of the flow cell shown in FIG. 2.
FIG. 5 illustrates an exploded view of the flow cell shown in FIG. 2.
FIG. 6 illustrates a bottom view of a piece of the flow cell shown in FIG. 2.
FIGS. 7A-C illustrate a number of cross-sections of the piece of the flow cell shown in FIG. 6.
FIG. 8 illustrates a top view of another piece of the flow cell shown in FIG. 2.
FIG. 9 illustrates a cross-sectional view of the piece of the flow cell shown in FIG. 8.
FIG. 10 illustrates a perspective view of a flow cell holder according to an embodiment.
FIG. 11A illustrates a side view of the flow cell holder with a clamping mechanism closed.
FIG. 11B illustrates a side view of the flow cell holder with a clamping mechanism open.
FIG. 12A illustrates a side view of portions of a clamping mechanism and an illumination system according to an embodiment.
FIG. 12B illustrates a cross-sectional view of the flow cell holder of FIG. 10 that includes a clamping mechanism and an illumination system.
FIG. 13 illustrates a plate of a clamping mechanism according to an embodiment.
FIG. 14 illustrates plates of a clamping mechanism and a flow cell according to an embodiment.
FIG. 15 illustrates a second embodiment of a flow cell according to a different embodiment.
FIG. 16 illustrates an exploded view of the flow cell shown in FIG. 15.
FIG. 17 illustrates a top view of the flow cell shown in FIG. 15.
FIG. 18 illustrates a flow chart of a process of reducing pathogens in a fluid according to an embodiment.
FIG. 19 illustrates a block diagram of a basic computer that may be used to implement embodiments.
FIG. 20 illustrates a graph of Log Reduction v. Dwell Time.
FIG. 21 illustrates a graph of Log Reduction v. Flow rate.
FIG. 22 illustrates a graph of Plasma/Riboflavin 50% Transmittance Distance.
FIG. 23 illustrates a graph of Acrylic UVT Transmittance.
FIG. 24 illustrates a graph showing log reduction as a function of depth for a predetermined flow rate.

## Detailed Description

The principles of the present invention may be further understood by reference to the following detailed description and the embodiments depicted in the accompanying drawings. It should be understood that although specific features are shown and described below with respect to detailed embodiments, the present invention is defined by the appended claims, and not limited to the embodiments described below or shown in the drawings. It is noted that several embodiments are described with respect to reducing pathogens in whole blood or blood components (e.g., plasma, platelets, red blood cells, leukocytes, buffy coat, or combinations thereof). However, the present invention is not limited to use with any particular fluid. Rather, the specific embodiments may be implemented with other fluids including biological fluids, non-biological fluids, or combinations thereof.

Reference will now be made in detail to the embodiments illustrated in the accompanying drawings and described below. Wherever possible, the same reference numbers are used in the drawings and the description to refer to the same or similar parts.

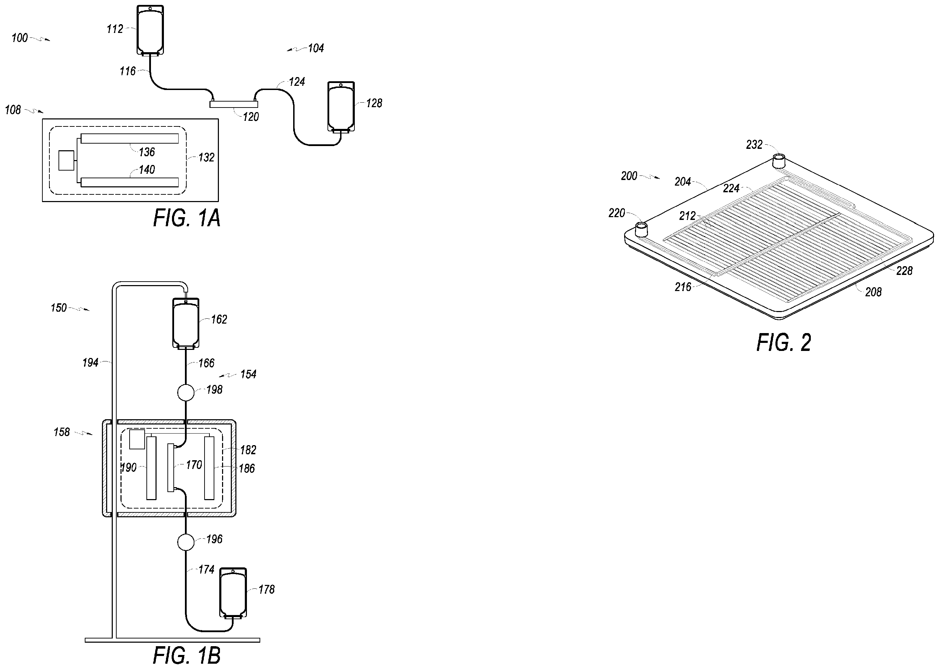

FIG. 1A illustrates an embodiment of a system 100 that may be used to reduce pathogens in a fluid, according to embodiments. The system 100 includes a flow cell system 104 and a flow cell holder 108. As described in greater detail below, the flow cell system 104 may work with flow cell holder 108 to reduce pathogens in a fluid.

Flow cell system 104 includes a first container (e.g., bag 112) which in embodiments contains the fluid to be pathogen reduced. Bag 112 is connected to flow cell 120 through tubing 116. Tubing 116 creates a fluid communication path from the bag 112 to the flow cell 120. Flow cell 120 is connected to a second container (e.g., bag 128) through tubing 124, which creates a fluid communication path between flow cell 120 and bag 128.

Flow cell holder 108 includes an illumination system 132. In the embodiment shown in FIG. 1A, illumination system 132 includes two light sources 136 and 140. As described in greater detail below, flow cell system 104 may be used with illumination system 132 to reduce pathogens in a fluid. In some embodiments, flow cell system 104 may be implemented as a disposable that is used in reducing pathogens in a single volume of fluid and then replaced.

FIG. 1B illustrates another flow-through pathogen reduction system 150 according to another embodiment. As shown in FIG. 1B, system 150 illustrates a flow cell system 154 and a flow cell holder 158. Flow cell system 154 includes a first container (e.g., bag 162) which in embodiments contains the fluid to be pathogen reduced, for example, blood or a blood component (e.g., red blood cells, plasma, platelets, buffy coat, leukocytes, or combinations thereof). Bag 162 is connected to flow cell 170 through tubing 168, which creates a fluid communication path from the bag 162 to the flow cell 170. Flow cell 170 is connected to a second container (e.g., bag 178) through tubing 174, which creates a fluid communication path between flow cell 170 and bag 178.

As shown in FIG. 1B, flow cell 170 has been positioned in flow cell holder 158, which includes an illumination system 182. Flow cell 170 has been positioned between the two light sources 186 and 190. In embodiments, the light source 186 and light source 190 are configured to illuminate flow cell 170 from at least two directions during a process of pathogen reducing a fluid. In embodiments, flow cell holder 158 may be configured with features to hold flow cell 170 but allow flow cell 170 to be removed after a pathogen reduction process has been completed. Some non-limiting examples of features include clips, rails, shelves, biased members, springs, sliding members, locks, etc.

System 150 also includes a stand 194. Stand 194 may include a base and a pole.

In operation, a user may begin a pathogen reduction process by hanging bag 162 from a pole of stand 194. Bag 162 may in embodiments contain a fluid to be pathogen reduced. For example, the fluid may be whole blood or a blood component (e.g., red blood cells, plasma, platelets, buffy coat, leukocytes, or combinations thereof). As disclosed below, in some embodiments, the fluid may also contain an additional material, e.g., a photosensitizer, that aids in the pathogen reduction process. The user may then position flow cell 170 in flow cell holder 158, between light source 186 and light source 190.

The light sources 186 and 190 may be activated to illuminate flow cell 170 from at least two directions. A fluid flow control device 198 may be activated, e.g., opened, to allow fluid to flow from bag 162 into flow cell 170. In embodiments, fluid flow control device 198 may be one or more of a clip, clamp, a frangible, a pump or combinations thereof. In other embodiments, there may be more than one fluid flow control device, e.g., fluid flow control device 196 which is located in a different location, e.g., such as along tubing 174. In embodiments, fluid flow control device 196 may be a pump that creates negative pressure in flow cell 170 drawing fluid through the flow cell 170.

In yet other embodiments, a fluid flow control device may be located on both tubing 168 and tubing 174 (e.g., 198 and 196). A user would activate both fluid flow control devices to allow fluid to flow from bag 162 into flow cell 170. In some embodiments, the fluid flow control devices 196 and 198 may be activated individually, e.g., 196 ON and 198 OFF; or 196 OFF and 198 ON.

As the fluid flows through the flow cell 170, the fluid may be illuminated by light sources 186 and 190 causing a reduction in pathogens. After pathogen reduction, the fluid may flow from flow cell 170 into bag 178 for storage.

In embodiments, the light sources 186 and 190 may radiate light of a particular wavelength that provides a pathogen reducing effect. For example, light sources 186 and 190 may radiate light in the ultraviolet spectrum such as light with a wavelength of between about 100 nm and about 400 nm. Some embodiments provide for use of light sources that radiate ultraviolet light within more specific ranges. As some non-limiting examples, some embodiments may utilize light sources that radiate UVA (wavelengths from about 315 nm to about 400 nm), UVB (wavelengths from about 280 nm to about 315 nm) and/or UVC (wavelengths from about 100 nm to about 280 nm). Without being bound by theory, it is believed that the energy from the ultraviolet light may destroy nucleic acids and disrupt DNA, which may interfere with cellular processes of microorganisms. As a result, pathogens, such as viruses and bacteria die. Ultraviolet light is merely one example. Other non-limiting examples of possible wavelengths of light that may be used include, visible light such as violet light (wavelengths from about 400 nm to about 420 nm), indigo light (wavelengths from about 420 nm to about 440 nm), blue light (wavelengths from about 440 nm to about 490 nm), and green light (wavelengths from about 490 nm to about 570 nm). In embodiments, light sources 186 and/or 190 may radiate light in any of the ranges noted above or in any combination of the ranges.

In other embodiments, in addition to light, the fluid may contain an additional material, e.g., a photosensitizer that aids in the pathogen reduction. Without being bound by theory, it is believed that photosensitizers include molecules that may be activated by light energy (e.g., ultraviolet light). The photosensitizer (or reaction products resulting from the activation) may disrupt bonds in DNA. In pathogens, such as viruses and bacteria, the disruption may lead to the death of the pathogen, or an inability to reproduce. Some non-limiting examples of photosensitizers that may be used in some embodiments include: porphyrins, flavins (e.g., riboflavin), psrolens, and combinations thereof.

FIGS. 2-9 illustrate various views of a flow cell 200 and pieces of flow cell 200 according to some embodiments. As shown in FIG. 2, flow cell 200 includes a first piece 204 and a second piece 208. Flow cell 200 also includes a plurality of channels 212, an inlet manifold 216, an inlet port 220, outlet manifolds 224 and 228, and an outlet port 232.

Referring to FIG. 2, in operation, a fluid to be pathogen reduced may be introduced into flow cell 200 through inlet port 220. The fluid may flow into inlet manifold 216 and into the plurality of channel 212. After flowing through the plurality of channels 212, the fluid flows into outlet manifolds 224 and 228. Finally, the fluid flows out of flow cell 200 through outlet port 232. In embodiments, the fluid may be exposed to light energy throughout the process of flowing through flow cell 200. In other embodiments, the fluid may be exposed to light energy only when passing through channels 212. As described in greater detail below, the features of flow cell 200 provide for exposing fluid to light energy (and in some embodiments to a photosensitive material), which reduces pathogens in the fluid. In embodiments, flow cell 200 is designed to ensure that fluid processed through flow cell 200 has a threshold amount of exposure to light energy to reduce pathogens by a predetermined amount. In other words, the fluid is provided with a minimum dose of light energy to reduce pathogens in the fluid by a predetermined amount. In embodiments, the process may result in a log reduction of about 1, about 1.5, about 2, about 2.5, about 3, about 3.5, about 4, about 4.5, about 5, about 5.5, or even about 6.0.

Also, embodiments may be designed to avoid exceeding a maximum threshold amount of exposure to light energy. That is, if the fluid is exposed to an amount of light energy above a threshold amount, other components of the fluid may be negatively affected. For example, too much energy may denature proteins that are desired to be maintained in the fluid.

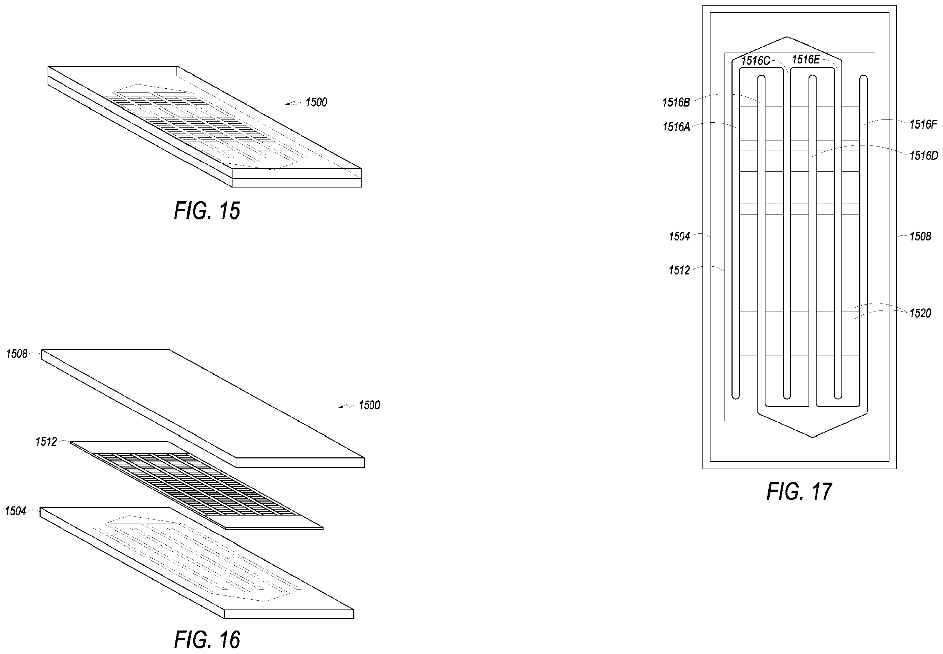

As noted above, flow cell 200 is shown as constructed from two pieces. This is merely one example of how a flow cell may be constructed according to embodiments. In other embodiments, flow cell 200 may be constructed from one piece or more than two pieces. For example, FIGS. 15-17 illustrate an embodiment of a flow cell that is constructed from three pieces.

Referring back to flow cell 200, the first piece 204 and second piece 208 may be made from materials that are transparent to the light used in processing the fluid, e.g., ultraviolet and/or visible light. For example, the first piece 204 and second piece 208 may be made from polymers, glass, ceramics, composites, or combinations thereof. In some embodiments, the first piece 204 and second piece 208 are made from a polymeric material that is transparent to a predetermined wavelength of light (e.g., ultraviolet, violet, indigo, blue, green, etc.).

Examples of polymeric materials that may be used in some embodiments include, but are not limited, to acrylics and polycarbonates. In embodiments, both pieces 204 and 208 may be made from the same, or similar, material. In other embodiments, pieces 204 and 208 may be made from different materials. In one embodiment, first piece 204 and second piece 208 may be made from a polymeric material (e.g., acrylic) that is transparent to ultraviolet light with wavelengths less than or equal to 320 nm. In another embodiment, first piece 204 and second piece 208 may be made from a polymeric material (e.g., acrylic).

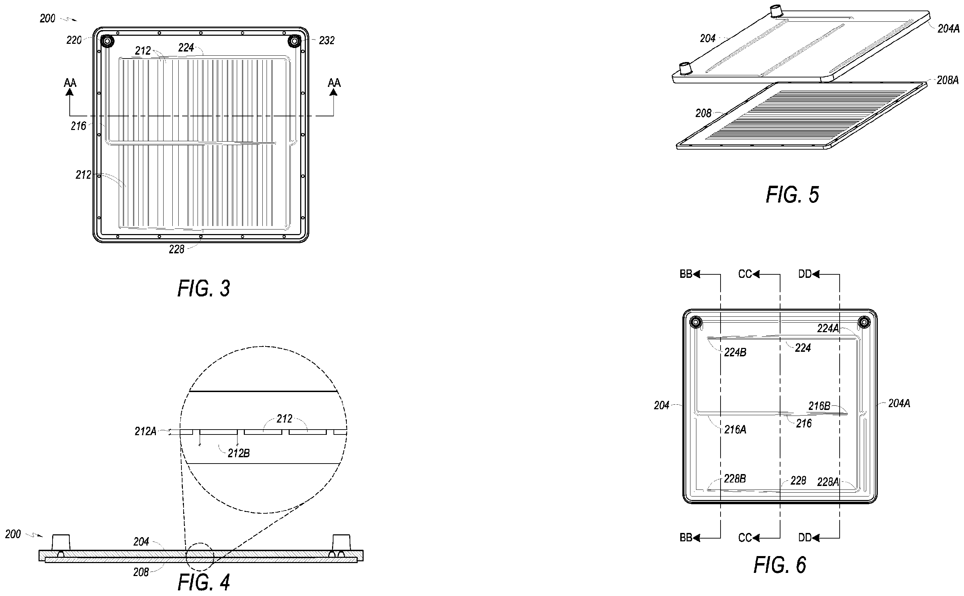

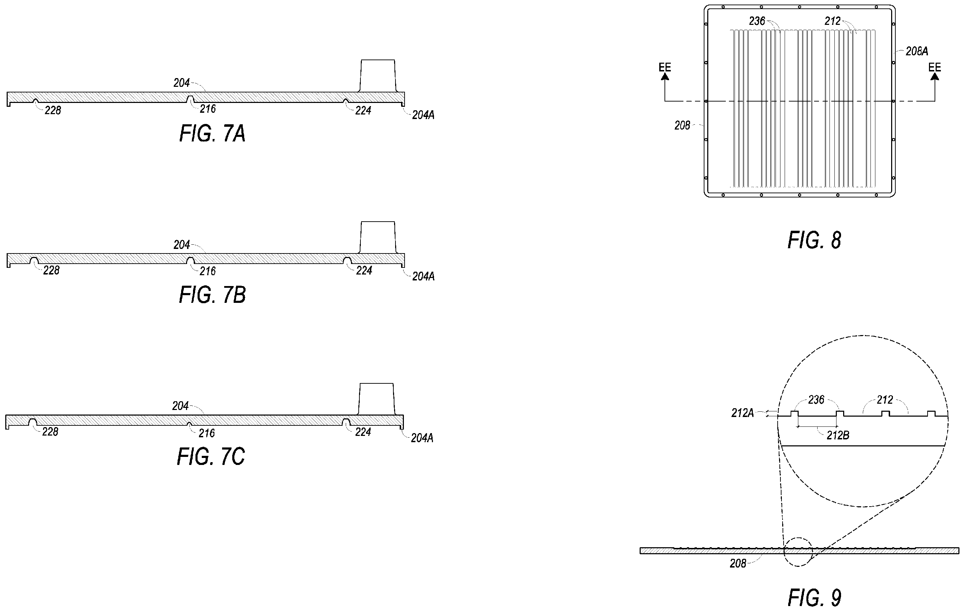

As shown in FIG. 2, first piece 204 and second piece 208 may be attached to create flow cell 200. In some embodiments, pieces 204 and 208 may be attached around their perimeters. That is, a portion of piece 204 around perimeter 204A (FIG. 5) may be attached to a portion of piece 208 around perimeter 208A (FIG. 8), such as for example by an adhesive, solvent welding, RF welding, ultrasonic welding, laser welding, etc. In some embodiments, it may be that only the perimeter of pieces 204 and 208 are attached, and no portions of the interior of pieces 204 and 208 are attached together. In these embodiments, a clamping mechanism, as described below, maybe used to apply pressure to flow cell 200 to push piece 204 and piece 208 together particularly in the interior where the two pieces may not be attached. The pressure aids in maintaining the dimension of the channels 212.

Referring now to FIG. 3, the plurality of channels creates fluid communication between inlet manifold 216 and outlet manifolds 224 and 228. In embodiments, the plurality of channels 212 may have characteristics that aid in reducing pathogens in a fluid.

FIG. 4 illustrates a cross-section of flow cell 200 taken along line AA-AA (FIG. 3). As shown in FIG. 4 each of the plurality of channels 212 include a depth 212A and a width 212B. In embodiments, the depth 212A of the channels 212 may be one of a plurality of parameters (e.g., depth, flow rate, energy dose, etc.) that is selected to ensure that the fluid being pathogen reduced is exposed to the necessary dose of light energy to provide a predetermined log reduction of at least one pathogen.

In embodiments depth 212A may be selected based on the fluid that may be processed. Without being bound by theory, it is believed that to reduce pathogens effectively using light energy, the light should penetrate through a depth of the fluid being pathogen reduced and expose the fluid to a minimum dose of light energy. As can be appreciated, the penetration of the light may depend, among other things, on the type of fluid, fluid transmissivity, and the thickness of the fluid, e.g., the depth of channels through which the fluid flows (212A). Additionally, the dose of light energy that a fluid receives may be affected by the amount of time that the fluid is exposed to the light energy. In other words, how much time the fluid may spend in a zone where it is exposed to the light energy, e.g., a flow rate of the fluid.

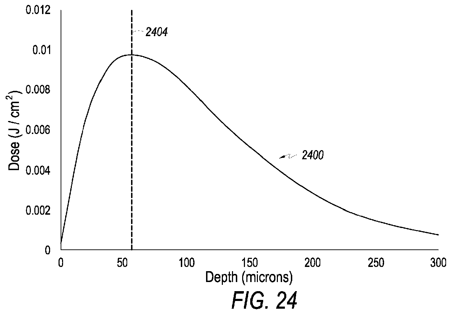

FIG. 24 illustrates a graph 2400 showing a possible relationship between minimum energy dose and depth size that may affect the pathogen reduction of a fluid flowing through a flow cell, e.g., flow cell 212. By minimum energy dose, it is meant the total amount of energy exposure (per area) received by a fluid element located at the midpoint of the channel depth. In the embodiment shown in FIG. 24, the fluid may be flowing at a predetermined flow rate, e.g., 10 ml/min. Without being bound by theory, it is believed that at small depth sizes, the fluid element may flow through the channel too quickly, e.g., at high velocity. That is, the amount of time (e.g., dwell time) spent in the zone where it is exposed to light is too short for the fluid element to receive much energy exposure. As the depth size increases, the velocity decreases, which increases the dwell time and consequently the amount of light energy that reaches fluid element. As the depth increases further, the amount of light energy transmitted through to the midpoint of the channel depth decreases exponentially. Accordingly, even though the dwell time may increase, the amount of light energy exposure received by the fluid element begins to decrease. As shown in FIG. 24, there may be an optimal depth 2404 at which the maximum amount of light energy may reach a fluid element located at the midpoint to effect the pathogen reduction. In embodiments, the optimal depth 2404 is selected as the depth 212A of channels 212.

In some embodiments, where the fluid to be pathogen reduced includes red blood cells (whole blood or packed red cells), the depth 212A (FIG. 4) may be selected based on a predetermined range of flow rates and a minimum dose to achieve a predetermined pathogen reduction (e.g., log reduction). In these embodiments, the depth 212A may be between about 0.025 mm and about 0.200 mm, such as between about 0.05 mm and about 0.175 mm, between about 0.075 mm and about 0.150 mm, or even between about 0.100 mm and about 0.125 mm. In embodiments depth 212A may be less than about 0.200 mm, less than about 0.175 mm, less than about 0.150 mm, less than about 0.125 mm, less than about 0.100 mm, or even less than about 0.075 mm. In other embodiments, depth 212A may be greater than about 0.025 mm, greater than about 0.05 mm, greater than about 0.075 mm, greater than about 0.100 mm, or even greater than about 0.125 mm. In embodiments, where fluid to be pathogen reduced includes whole blood, the depth 212A may be between about 0.075 mm and about 0.150 mm or even between about 0.100 mm and about 0.125 mm. In embodiments, where fluid to be pathogen reduced includes packed red blood cells, the depth 212A may be between about 0.0125 mm and about 0.100 mm or even between about 0.025 mm and about 0.075 mm.

In some embodiments, where the fluid to be pathogen reduced includes platelets and/or plasma, the depth 212A may be selected based on a predetermined range of flow rates and a minimum dose to achieve a predetermined pathogen reduction (e.g., log reduction). In these embodiments, the depth 212A may be between about 0.150 mm and about 2 mm, such as between about 0.175 mm and about 1.5 mm, between about 0.200 mm and about 1 mm, or even between about 0.225 mm and about 0.500 mm. In embodiments depth 212A may be less than about 2 mm, less than about 1.75 mm, less than about 1.5 mm, less than about 1.25 mm, less than about 1 mm, less than about 0.75 mm, less than about 0.50 mm, less than about 0.400 mm, less than about 0.300 mm or even less than about 0.200 mm. In other embodiments, depth 212A may be greater than about 0.100 mm, greater than about 0.200 mm, greater than about 0.300 mm, greater than about 0.400 mm, greater than about 0.500 mm, greater than about 0.600 mm, greater than about 0.700 mm, greater than about 0.800 mm, greater than about 0.900 mm, greater than about 1.00 mm, or even greater than about 1.500 mm. In embodiments, where fluid to be pathogen reduced includes plasma and/or platelets, the depth 212A may be between about 0.150 mm and about 2 mm or even between about 0.200 mm and about 1.5 mm.

In other embodiments, width 212B may be selected to increase the flow rate of fluid that may be processed through a flow cell. In other words, the larger the width 212B, the larger the flow rate of fluid through flow cell 200. In embodiments, the width 212B may be between about 1 mm and about 20 mm, between about 1.25 mm and about 17.5 mm, between about 1.5 mm and about 15 mm, between about 1.75 mm and about 12.5 mm, or even between about 2.0 mm and about 10 mm. In embodiments, the width 212B may be greater than about 0.5 mm, greater than about 1 mm, greater than about 1.5 mm, greater than about 2.0 mm, or even greater than about 2.5 mm. In other embodiments, width 212B may be may be less than about 25 mm, less than about 20 mm, less than about 15 mm, less than about 10 mm, or even less than about 5 mm.

In embodiments, the width 212B may be no wider than a predetermined ratio of width 212B to depth 212A. For example, in embodiments the ratio of width 212B to depth 212A may be less than about 150, about 125, about 100, about 75, about 50, about 25, about 20, about 15 or even less than about 10. In other embodiments, the ratio of width 212B to depth 212A may be greater than about 2, than about 3, than about 4, or even greater than about 5.

In some embodiments, to increase flow rate of fluid through flow cell 200, instead of having width 212B larger, flow cell 200 may in embodiments include more channels 212. In some embodiments, flow cell 200 may include more than about 20 channels, about 30 channels, about 40 channels, about 50 channels, about 60 channels, about 70 channels, about 80 channels, about 90 channels, or even more than about 100 channels. In some embodiments, flow cell 200 may include less than about 1000 channels, about 900 channels, about 800 channels, about 700 channels, about 600 channels, or even less than about 500 channels.

As noted above, and shown in FIG. 5, flow cell 200 may be constructed, in embodiments, by attaching first piece 204 and second piece 208. Each of pieces 204 and 208 may include different features that when attached to each other forms the structure of flow cell 200, as described above. FIGS. 6-9 illustrate various views of pieces 204 and 208.

FIG. 6 illustrates a bottom view of piece 204 of flow cell 200. As shown in FIG. 6, piece 204 may include channels that create manifolds 216, 224, and 228 in flow cell 200. Manifolds 216, 224, and 228, in embodiments, have particular structural features that aid in the flow of fluid through flow cell 200.

In embodiments, flow cell 200 may include features that reduce the pressure drop as fluid flows through flow cell 200. A drop in pressure may create short circuit situations in which the fluid does not flow through all of the channels, but rather flows only through a few of the first channels where fluid begins to flow. Also, the features may address the possible issue of stagnant fluid being over exposed to light energy.

Some of these features (of the manifolds) include the shape and structure of manifolds 216, 224, and 228. As can be seen in FIGS. 6-7C, manifolds 216, 224, and 228 have a tapered structure. That is, the cross-sectional area of the manifold is larger in some locations and gets smaller along a length of the manifold.

For example, manifold 216 has a larger cross-sectional area at a proximal end 216A than at a distal end 216B. FIG.7A illustrates a cross-section of piece 204 taken along line BB-BB (FIG. 6). FIG.7B illustrates a cross-section of piece 204 taken along line CC-CC (FIG. 6). FIG.7C illustrates a cross-section of piece 204 taken along line DD-DD (FIG. 6). As shown in FIGS. 7A-7C, as cross-sectional areas are taken from the proximal end 216A to the distal end 216B along the length of manifold 216, the cross-sectional areas get progressively smaller.

Similarly, manifold 224 has a proximal end 224A and a distal end 224B, and manifold 228 has a proximal end 228A and a distal end 228B. As shown in FIG. 6, manifolds 224 and 228 have their distal ends 224B and 228B on a same side of piece 204 as the proximal end 216A of manifold 216. As shown in FIGS. 7A-7C, as cross-sectional areas are taken from the distal ends 224B and 228B to the proximal ends 224A and 224B along the length of manifolds 224, the cross-sectional areas get progressively larger.

It is noted that although FIGS. 7A-7C, illustrate the cross-sections of manifolds 216, 224, and 228 in particular shapes, other embodiments may have different cross-sectional shapes. As some non-limiting examples, the manifolds 216, 224, and 228 may have square, rectangular, triangular, elliptical, diamond, or other cross-sectional shape.

In addition to features of the actual manifold, flow cell 200 may also utilize other features to manage pressure within the flow cell 200. For example, the number of manifolds, the pattern of the manifolds (i.e., locations), lengths of manifolds, lengths of channels, may all be modified to control pressure drop and fluid flow in flow cell 200.

FIG. 8 illustrates a top view of piece 208 of flow cell 200. As shown in FIG. 8, piece 208 includes features, namely a plurality of walls 236 that create channels 212 in flow cell 200. FIG. 9 illustrates a cross-sectional view of piece 208 taken along line EE-EE (FIG. 8). As illustrated in FIGS. 8 and 9, the number, width, and depth of channels 212 may be created by the dimensions of walls 232.

Referring again to FIG. 3, when pieces 204 and 208 are attached, e.g., along their perimeters 204A and 204B for example, the structures (including manifolds 216, 224, and 228; channels 212) of flow cell 200 are created. As shown in FIG. 3, walls 236 create channels 212 between manifold 216 and 224 and between manifold 216 and 228. As illustrated in FIG. 3, at least one of the plurality of channels 212 creates fluid communication between the proximal end 216A of manifold 216 and the distal end 224B of manifold 224. Similarly, at least a second one of the plurality of channels 212 creates fluid communication between the proximal end 216A of manifold 216 and the distal end 228B of manifold 228. In some embodiments, only the perimeters 204A and 208A are attached and the interiors of pieces 204 and 208 are not attached (e.g., bonded) together.

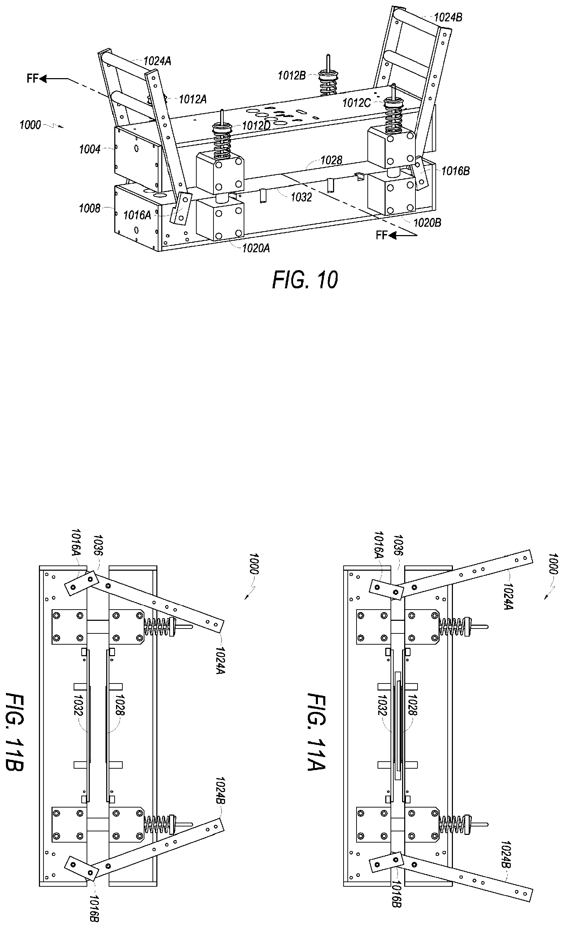

FIG. 10 illustrates a perspective view of a flow cell holder 1000 according to an embodiment. Flow cell holder 1000 may be configured to hold a flow cell (e.g., flow cell 200) during illumination of the flow cell while fluid is flowing through the flow cell. As described below, flow cell holder 1000 may in embodiments include other features/mechanisms to aid in the pathogen reduction process.

Flow cell holder 1000 may include a first portion 1004 and a second portion 1008. Flow cell holder 1000 may also include features of a clamping mechanism that allows first portion 1004 and/or second portion 1008 to move to create a space between first portion 1004 and second portion 1008. The clamping mechanism may also allow first portion 1004 and/or second portion 1008 to move to clamp a flow cell between the first portion 1004 and the second portion 1008.

The clamping mechanism may include a number of structures. For example, the clamping mechanism may include springs 1012A-D, hinges 1016A & 1016B, alignment blocks 1020A & 1020B, handles 1024A & 1024B, and plates 1028 and 1032.

FIG. 11A illustrates a side view of the flow cell holder 1000 with a clamping mechanism in a closed position. FIG. 11B illustrates flow a side view of the flow cell holder with a clamping mechanism in an open position. As illustrated in FIG. 11B, when the clamping mechanism is in an open position, plates 1028 and 1032 have a space 1036 between them. A flow cell (e.g., flow cell 200) may be positioned in space 1036. FIG. 11B illustrates the clamping mechanism in an open position with space 1036, reduced or eliminated. As noted above, a flow cell may be clamped between plates 1028 and 1032 when the clamping mechanism is in the closed position (FIG. 11A).

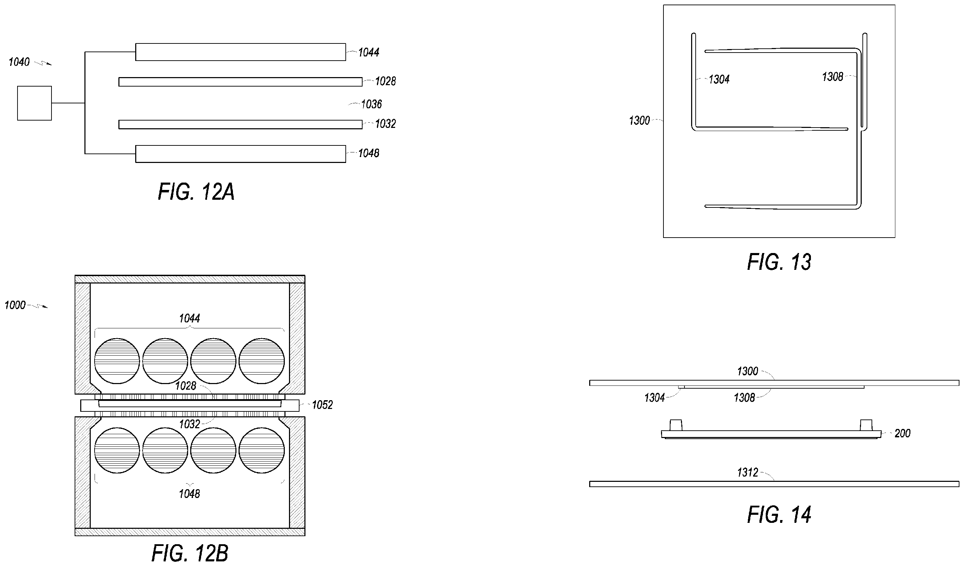

FIG. 12A illustrates portions of a clamping mechanism, namely plates 1028 and 1032 (and space 1036 between plates 1028 and 1032) and portions of an illumination system 1040, namely light source 1044 and light source 1048 according to an embodiment. FIG. 12A illustrates the interaction and spatial relationship between plates 1028 and 1032 and light sources 1044 and 1048.

As shown in FIG. 12A, plate 1028 is opposite plate 1032 and as noted above, the plates 1028 and 1032 are configured to hold a flow cell between them. As also shown, light source 1044 is adjacent plate 1028 and light source 1048 is adjacent plate 1032. In embodiments, plates 1028 and 1032 are transparent to the light emitted by light sources 1044 and 1048. As can be appreciated, in a pathogen reduction process, a flow cell may be clamped between plates 1028 and 1032. When light sources 1044 and 1048 are activated, the flow cell may be illuminated from more than one direction, namely from the top by light source 1044 and the bottom by light source 1048.

FIG. 12B illustrates a cross-sectional view of flow cell holder 1000 taken along line FF-FF. FIG. 12B illustrates the clamping mechanism in a closed position and holding a flow cell 1052 between plates 1028 and 1032. As shown in FIG. 12B, light sources 1044 and 1048 may be implemented using a plurality of bulbs. This is merely for illustrative purposes and embodiments may implement light sources 1044 and 1048 using any type of illumination device(s) non-limiting examples including: LEDs, incandescent bulbs, fluorescent bulbs, halogen bulbs, xenon bulbs, and/or combinations thereof.

As noted above with respect to FIG. 1, embodiments provide for light sources to emit light with wavelengths that have a pathogen reducing effect, which may include ultraviolet light. It is noted that in addition to illumination devices, light sources 1044 and 1048 may have additional components that aid in the illumination of flow cell 1052. For example, light sources 1044 and 1048 may include components such as: filters, wave guides, lenses, mirrors and/or combinations thereof.

As noted above, plates 1028 and 1032 are transparent to at least a predetermined wavelength of light e.g., ultraviolet light, violet light, indigo light, blue light, and/or green light. By transparent, it is meant that at least about 85 % of light of a particular wavelength is transmitted through plates 1028 and/or 1032. In embodiments, greater than about 90 %, greater than about 95 % or even greater than about 98 % of light of a particular wavelength may be transmitted through plates 1028 and/or 1032.

In embodiments, plates 1028 and 1032 may be made from any suitable material that has transparency to a particular wavelength of light. Further, because the plates 1028 and 1032 may clamp down on a flow cell, they may be made from materials with the necessary structural integrity to withstand pressure used in clamping the flow cell without failing (e.g., fracturing). For example, plates 1028 and 1032 may be made from polymers, glass, ceramics, composites, or combinations thereof. In some embodiments, plates 1028 and 1032 may be made from a glass material that is transparent to the predetermined wavelength of light. Some non-limiting examples of glass that may be used include: fused quartz, borosilicate glass, and soda-lime glass. In other embodiments, polymeric materials may be used. Non-limiting examples of polymers that may be used include acrylics and polycarbonates. In embodiments, both plates 1028 and 1032 may be made from the same material. In other embodiments, both plates 1028 and 1032 may be made from different materials.

In addition to materials properties, plates 1028 and 1032 may also include other structural features. FIG. 13 illustrates a plate 1300 which may be part of a clamping mechanism according to an embodiment. Plate 1300 includes gaskets 1304 and 1308. In embodiments, when plate 1300 clamps a flow cell, gaskets 1304 and 1308 may contact the flow cell.

Gaskets 1304 and 1308 may provide a number of functionalities. For example, in some embodiments, the gaskets 1304 and 1308 may provide cushion when clamping the flow cell. In other embodiments, gaskets 1304 and 1308 may, in addition to other functions, aid in holding the flow cell and preventing the flow cell from moving during a pathogen reduction process.

In other embodiments, the gaskets 1304 and 1308 may be located on plate 1300 to correspond to edges (e.g., portions of a perimeter) of the flow cell, holding the flow cell in place. In other embodiments, gaskets 1304 and 1308 may be made of materials (e.g., polymer, rubber, and/or combinations thereof) that grip the flow cell to hold it in place. In some embodiments, the material may be somewhat transparent to a particular wavelength of light in order not to interfere with the pathogen reduction process.

In the embodiment shown in FIG. 13, gaskets 1304 and 1308 are shaped to correspond to manifolds; for example, manifolds 216, 224, and 228 of flow cell 200. The gaskets may be designed to align with manifolds 216, 224, and 228 when plate 1300 clamps flow cell 200. In some embodiments, gaskets 1304 and 1308 may be somewhat transparent to a particular wavelength of light in order not to interfere with the pathogen reduction process. In other embodiments, gaskets 1304 and 1308 may not be transparent to a particular wavelength of light but not affect the pathogen reduction process because the necessary light energy dose may be provided while flowing through the channels (e.g., channels 212) of the flow cell. In other embodiments, the gaskets may be designed to intentionally shield portions of the flow cell from the light to avoid overexposing the fluid to light energy.

In other embodiments, the gasket that are not attached to plate 1300 may be used. For example, in some embodiments, the gaskets may be a separate piece (or pieces). In these embodiments, the gaskets may be positioned between plates and a flow cell. The gaskets may include a relatively soft polymeric material (e.g. rubber) attached to a frame, which itself may be made from a polymer, metal, and/or a composite material. These are merely examples provided for illustrative purposes.

FIG. 14 illustrates an exploded view of a clamping mechanism that includes plate 1300. As shown in FIG. 14, portions of gaskets 1304 and 1308 may be aligned with flow cell 200. When flow cell 200 is clamped, e.g., in contact with gaskets 1304 and 1308 and plate 1312, gaskets 1304 and 1308 may be aligned with specific features of flow cell 200, such as manifolds 216, 224, and/or 228. In embodiments, having gaskets 1304 and 1308 may allow additional pressure to be used when clamping flow cell 200. As described above, in embodiments flow cell 200 may be constructed by two pieces that may be attached along their perimeter. Having the clamping mechanism maintain a moderate amount of pressure on the flow cell 200 may aid in maintaining the channels 212 dimensions during fluid flow.

FIGS. 15-17 illustrate views of a flow cell 1500 according to another embodiment. As shown in FIGS. 15 and 16, flow cell 1500 is constructed from three pieces. Piece 1504 which may include a number of pathways that create manifolds 1516A-D. Piece 1508 may be a top piece positioned above piece 1504. Piece 1512 may be positioned between piece 1504 and 1508 and provide walls that create a plurality of channels 1520. The plurality of channels 1520 may create fluid communication between one or more manifolds 1516A-F. Similar to flow cell 200 (FIG. 2) fluid flowing through flow cell 1500 may be illuminated with light energy to pathogen reduce the fluid.

Pieces 1504 and 1508 may be made from materials that are transparent to a predetermined wavelength of light. For example, pieces 1504 and 1508 may be made from materials such as polymers, glass, ceramics, composites, or combinations thereof. In some embodiments, pieces 1504 and/or 1508 may be made from a glass material that is transparent to the predetermined wavelength of light. Some non-limiting examples of glass that may be used include: fused quartz, borosilicate glass, and soda-lime glass. In other embodiments, polymeric materials may be used. Non-limiting examples of polymers that may be used include acrylics and polycarbonates. In embodiments, pieces 1504 and 1508 may be made from the same type of material. In other embodiments, pieces 1504 and 1508 may be made from different materials.

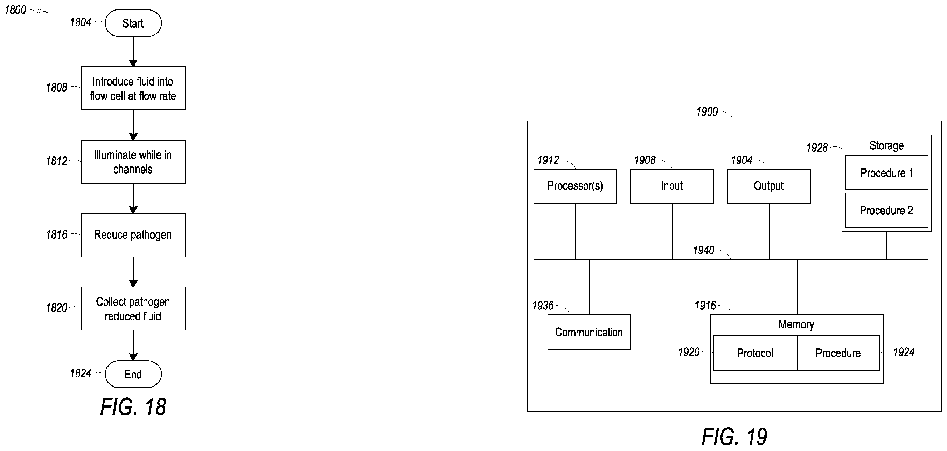

FIG. 18 illustrates a flow chart 1800 of a process of reducing pathogens in a fluid according to an embodiment. Although features of flow cells (e.g., flow cells 200 and/or 1500), flow cell holders (e.g., flow cell holder 1000), clamping mechanisms (e.g., plates 1028 and 1032) and/or systems (e.g., system 100) may be described as part of performance of the steps of the flow chart 1800, embodiments are not limited thereto. Indeed, other types of flow cells, holders, clamping mechanisms, and/or systems may be used (with different structures) in the process of flow chart 1800.

Flow chart 1800 starts at 1804, and passes to step 1808 where a fluid to be pathogen reduced is introduced into a flow cell at a flow rate. In embodiments, the flow cell may include a plurality of channels with a depth and a width. In one embodiment, the flow cell is similar to flow cell 200 noted above. The plurality of channels may correspond to channels 212 which provide fluid communication between an inlet manifold and one or more outlet manifolds. In embodiments, the plurality of channels may be relatively straight channels that provide a direct line-of-sight between an inlet manifold and an outlet manifold. That is, the channels are not serpentine or curved channels. The channels may have the dimensions, depth and width, described above with respect to flow cell 200.

In embodiments, the flow rate may be selected to process a volume of fluid within a predetermined period of time. For example, in some embodiments, it may be desirable to process between about 5 liters and about 100 ml of the fluid through the flow cell in less than about 2 hours, less than about 1.5 hours, less than about 1 hour, less than about 0.5 hours, less than about 0.25 hours, or even less than about 0.125 hours. In these embodiments, the flow rate may be selected to be between about 0.5 ml/min and about 75 ml/min, such as between about 1 ml/min and about 50 ml/min. In embodiments, the flow rate may be greater than about 0.5 ml/min, about 0.75 ml/min, about 1 ml/min, about 1.5 ml/min, about 2 ml/min, about 2.5 ml/min, about 3.0 ml/min, about 3.5 ml/min, or even greater than about 4 ml/min. In other embodiments, the flow rate may be less than about 100 ml/min, about 90 ml/min, about 80 ml/min, about 70 ml/min, about 60 ml/min, about 50 ml/min, about 40 ml/min, about 30 ml/min, about 20 ml/min, or even less than about 10 ml/min. These flow rates may be selected in combination with one or more dimensions (e.g., depth, width, etc.) of channels 212, described above with respect to flow cell 200.

After step 1808, flow 1800 passes to step 1812, where the fluid is illuminated while it passes through the channels. In embodiments, the fluid may be illuminated from at least two directions. In some embodiments, step 1812 may be performed while the flow cell (e.g., flow cell 200) is in a cell holder, such as cell holder 1000. The cell holder may include a lighting system (e.g., system 132 or system 1040) that illuminates the flow cell and the fluid while the fluid flows through the plurality of channels. For example, the fluid may be illuminated from a top and a bottom of the plurality of channels.

Flow 1800 passes from step 1812 to step 1816, where the fluid is pathogen reduced. In embodiments, the reduction in pathogens may be effected by the illumination of the fluid as it passes through the channels. In some embodiments, the light alone may create the pathogen reducing effect. In other embodiments, an additional material, may work in combination with the light energy to effect the pathogen reduction. For example, a photosensitizer may be added to the fluid before or during step 1808. The photosensitizer may be activated when illuminated. The activated photosensitizer (or reaction products from the activation) result in disruption of the genetic material and death, or an inability to replicate, of the pathogen. Flow 1800 ends at 1824 where the pathogen reduced fluid is collected.

Although flow chart 1800 has been described with steps listed in a particular order, the embodiments are not limited thereto. In other embodiments, steps may be performed in different order, in parallel, or any different number of times, e.g., before and after another step. Also, flow chart 1800 may include some optional steps or substeps. However, those steps above that are not indicated as optional should not be considered as essential to the invention, but may be performed in some embodiments of the present invention and not in others.

FIG. 19 illustrates example components of a basic computer system 1900 upon which embodiments of the present invention may be implemented. Computer system 1900 may perform some steps in the methods for introducing fluid into a flow cell or illuminating fluid in a flow cell. System 1900 may be a controller for controlling features, e.g., flow control devices, pumps, valves, rotation of bioreactors, motors, lighting systems, clamping mechanisms etc., of systems such as systems 100, 1000, and/or 1040 shown above.

Computer system 1900 includes output device(s) 1904, and/or input device(s) 1908. Output device(s) 1904 may include one or more displays, including CRT, LCD, and/or plasma displays. Output device(s) 1904 may also include a printer, speaker, etc. Input device(s) 1908 may include a keyboard, touch input devices, a mouse, voice input device, etc.

Basic computer system 1900 may also include a processing unit 1912 and/or a memory 1916, according to embodiments of the present invention. The processing unit 1912 may be a general purpose processor operable to execute instructions stored in memory 1916. Processing unit 1912 may include a single processor or multiple processors, according to embodiments. Further, in embodiments, each processor may be a multi-core processor having one or more cores to read and execute separate instructions. The processors may include general purpose processors, application specific integrated circuits (ASICs), field programmable gate arrays (FPGAs), other integrated circuits.

The memory 1916 may include any tangible medium for short-term or long-term storage for data and/or processor executable instructions, according to embodiments. The memory 1916 may include, for example, Random Access Memory (RAM), Read-Only Memory (ROM), or Electrically Erasable Programmable Read-Only Memory (EEPROM). Other storage media may include, for example, CD-ROM, tape, digital versatile disks (DVD) or other optical storage, tape, magnetic disk storage, magnetic tape, other magnetic storage devices, etc. In embodiments, system 1900 may be used to control activation of a light source and/or various flow control devices, pumps, valves, etc. of a pathogen reducing system. Memory 1916 can store protocols 1920 and procedures 1924, such as protocols and procedures for introducing fluid into a flow cell and/or illuminating fluid in a flow cell, which would control operation of pumps, valves, clamping mechanisms, etc.

Storage 1928 may be any long-term data storage device or component. Storage 1920 may include one or more of the systems described in conjunction with memory 1916, according to embodiments. Storage 1928 may be permanent or removable. In embodiments, system 1900 is part of a system for reducing pathogens in a fluid and storage 1928 may store various procedures for utilizing the system to pathogen reduce a fluid.

EXAMPLES

Described below are some examples of embodiments. However, it is noted that although specific methods, apparatuses, and systems are described below, these are provided merely for illustrative purposes, and the present invention is not limited to the specific details in the examples below.

Example 1

This example provides information on the PhiX174 (pathogen) reduction in whole blood with riboflavin at three different flow rates in five different prototype flow-through flow cells.

This study is designed to obtain insight into the correlation between flow rate, channel thickness and pathogen reduction of whole blood. The flow cells in this study have thin channels and different flow configurations. Thin channels are used because whole blood is less transmissive than plasma.

The flow cell mounts in a fixture (e.g. a flow cell holder) which maintains channel thickness by applying pressure from both the top and bottom. The fixture houses eight illumination lamps driven at 800 mA with four on top and four on bottom producing an irradiance of 4.5 mW/cm$^2$. Product can be flowed through the flow-cell, which is mounted adjacent to the light source, and then samples can be taken on the back end. Whole blood combined with riboflavin and PhiX174 (pathogen) may be used as the test medium (product).

A peristaltic pump to control flow rate is used with the pump will located after the flow cell so that negative pressure is applied to the flow cell by the pump instead of positive pressure. The pump is calibrated prior to use to ensure flow rate accuracy.

Two flow cell configurations being tested may be referred to as "8p" and "4sx2p". The 8p configuration has all the channels in parallel; therefore the fluid crosses one channel before exiting the flow cell. The 4sx2p configuration requires the fluid to cross four channels in series before exiting the flow cell. All the flow cells have the same channel surface area regardless of channel configuration; therefore dwell time is only proportional to channel thickness and flow rate. See Table 1 and Table 2.

Table 1: Naming Convention

| Flow | Dwell Time (s) | | |
|---|---|---|---|
| Cell | 2 | 4 | 6 |
| A | A2 | A4 | A6 |
| B | B2 | B4 | B6 |
| C | C2 | C4 | C6 |
| D | D2 | D4 | D6 |
| E | E2 | E4 | E6 |

| Flow Cell | Spacer Thickness (Thousands of an inch) | Channel Configuration |
|---|---|---|
| A | 1 | 8p |
| B | 1 | 4sx2p |
| C | 2 | 8p |
| D | 2 | 4sx2p |
| E | 3 | 4sx2p |

Table 2: Flow Rates and Timing

*Flow Rates (mL/min)*

| Spacer Thickness | Flow Cell | Dwell Time (s) | | |
|---|---|---|---|---|
| | | 2 | 4 | 6 |
| 1 | A | 9.7 | 4.9 | 3.2 |
| | B | 9.7 | 4.9 | 3.2 |
| 2 | C | 19.5 | 9.7 | 6.5 |
| | D | 19.5 | 9.7 | 6.5 |
| 3 | E | 29.2 | 14.6 | 9.7 |

| Flow Rate (mL/min) | Purge Time (mm:ss) | Sample Time (mm:ss) |
|---|---|---|
| 3.2 | 12:30 | 04:41 |
| 4.9 | 08:10 | 03:04 |
| 6.5 | 06:09 | 02:18 |
| 9.7 | 04:07 | 01:33 |
| 14.6 | 02:44 | 01:02 |
| 19.5 | 02:03 | 00:46 |
| 29.2 | 01:22 | 00:31 |

## 1. PROTOCOL STEPS

### 1.1. Whole Blood Preparation

1.1.1. Obtain at least 1,100 mL of type matched whole blood; record the BUI number of the units used.

1.1.2. Pool the whole blood units into one bag.

1.1.3. Weigh the whole blood, including the bag, record on the DCS.

1.1.4. Add 4 mL of PhiX174 inoculum to the whole blood pool and record on the DCS.

1.1.5. Obtain one pouch of riboflavin (~35 mL) for every unit of whole blood used to create the pool.

1.1.6. Sterile connect each riboflavin pouch to the pool and drain the entire contents of the bag into the pool and document on the DCS. Purge all residual air into the empty riboflavin bags.

1.1.7. Aseptically remove three 5 mL samples from the whole blood pool and transfer into three sample tubes labeled "PRE". These samples will be used as hold controls for the study.

1.1.8. Measure the hematocrit of the whole blood pool following LOP-0032 and record in the DCS.

1.2. Pump Preparation

1.2.1. Load the pump calibration tubing supplied with the disposables into the pump head.

1.2.2. Place an empty beaker onto a scale next to the outlet side of the pump.

1.2.3. Place the inlet side of the calibration tubing into deionized water and the outlet side of the pump into the beaker on the scale.

1.2.4. Prime the pump.

1.2.5. Select L/S 13 as the tubing type and set the flow rate to 20 mL/min.

1.2.6. Tare the scale with the beaker on it.

1.2.7. Press CAL and note the calibration volume that appears.

1.2.8. Press STOP/START and the pump will dispense the calibration volume into the beaker.

1.2.9. Once the pump has stopped, enter the weight of calibration volume that was dispensed into the pump using the UP/DOWN arrow keys.

1.2.10. Press SIZE to exit the calibration cycle.

1.3. Disposable Preparation (In biosaftey cabinet)

1.3.1. Obtain sterile prototype flow-through disposable, and a sterile flow-cell assembly.

1.3.2. Close all clamps on the set.

1.3.3. Connect the flow-cell to the disposable components.

1.3.4. Connect the reservoir bag with the whole blood to the disposable components.

1.3.5. Connect a bag of saline to the disposable components.

1.4. Illumination Preparation

1.4.1. Label the first four sample bags with the corresponding sample name (Table 1). The fourth sample bag is intentionally not used and intended to be an extra in the event that an extra sample bag is required.

1.4.2. Mount the flow-cell and ensure the flow cell is properly loaded into the enclosure.

1.4.3. Open the clamps to allow flow from the saline bag to the waste bag. All other clamps should be closed.

1.4.4. Prime the flow-cell with saline with a flow rate equal to or less than the maximum sample flow rate.

1.4.5. Ensure all other clamps are closed.

1.4.6. Prepare the illumination setup.

1.4.7. Apply UV protective safety glasses to all personnel working in the area.

1.4.8. Ensure proper light barriers are in place to minimize UV light expose.

1.4.9. Turn on the UV lamps and cooling fan and allow 10 minutes for the lamp to warm up.

1.5. Sample Generation

1.5.1. Ensure the lamps are warmed up and have been running for 10 minutes.

1.5.2. Purge the system and take sample.

1.5.2.1. Ensure that the pump is programmed with the correct flow rate from Table 2.

1.5.2.2. Open all the clamps to allow flow from the plasma to the waste bag.

1.5.2.3. Turn on the pump and start the stop watch (approximately simultaneously).

1.5.2.4. Monitor fluid progress through the disposable.

1.5.2.5. After the purge time from Table 2 has elapsed (allowing at least 40 mL to of blood to flow into the waste bag) open the clamp to the corresponding sample bag then close the clamp to the waste bag.

1.5.2.6. After the sample time from Table 2 has elapsed (allowing at least 15 mL to of blood to flow into the sample bag) open the clamp to the waste bag then close the clamp to the sample bag.

1.5.3. Repeat from step 1.5.2 for each flow rate in Table 2.

1.5.4. Once all samples for a flow cell have been collected, turn off the lamps and purge the flow cell with saline to remove remaining blood.

1.5.5. Seal and remove all sample bags. Aseptically remove three 4 mL samples from each bag and transfer into three sample tubes labeled as indicated in Table 1.

1.5.6. Submit one sample tube for each sample for viral titer and save the other sample for later analysis.

1.5.7. Repeat from step 3.3 for each flow cell. Use the same blood pool for the entire study.

1.5.8. Aseptically remove three 4 mL samples from the blood pool and transfer into two sample tubes labeled "HC". These samples will be used as hold controls for the study.

1.6. Sample Handling

1.6.1. Once all samples have been collected, centrifuge them at 3000 RCF for 10 minutes.

1.6.2. Decant off the plasma supernatant into a labeled 2 mL cryovial.

1.6.3. Place the cryovials into an ultra-freezer (-80°C) for analysis at a later date.

Table 3 below summarizes the results of the study. Cells highlighted are samples that are beyond the limit of detection. Flow cells A and B may clog making some of the samples unobtainable. The data for flow cells C, D and E is included below (other than C6 which is at the limit of detection).

Table 3: Summary of Results

| Sample ID | PhiX174 Concentration (Log PFU/mL) | Log Reduction | Plasma Hemoglobin (g/dL) | % Hemolysis | Dwell Time (s) | Flow Rate (mL/min) | Cell Config | Channel Thickness |
|---|---|---|---|---|---|---|---|---|
| PRE | 7.11 | NA | 0.03 | 0.17% | NA | | | |
| HC | 6.79 | | 0.03 | 0.17% | | | | |
| A2 | **3.48** | 3.47 | 0.03 | 0.17% | 2 | 9.7 | 8p | 0.001" (0.0254 mm) |
| A4 | **3.48** | 3.47 | 0.2 | 1.16% | 4 | 4.9 | 8p | |
| B2 | 4.23 | 2.72 | 1.04 | 6.02% | 2 | 9.7 | 4sx2p | |
| C2 | 5.09 | 1.86 | 0.03 | 0.17% | 2 | 19.5 | 8p | 0.002" (0.0508 mm) |
| C4 | 3.87 | 3.08 | 0.03 | 0.17% | 4 | 9.7 | 8p | |
| C6 | **3.48** | 3.47 | 0.03 | 0.17% | 6 | 6.5 | 8p | |
| D2 | 5.48 | 1.47 | 0.04 | 0.23% | 2 | 19.5 | 4sx2p | |
| D4 | 4.6 | 2.35 | 0.04 | 0.23% | 4 | 9.7 | 4sx2p | |
| D6 | 3.79 | 3.16 | 0.03 | 0.17% | 6 | 6.5 | 4sx2p | |
| E2 | 5.68 | 1.27 | 0.04 | 0.23% | 2 | 29.2 | 4sx2p | 0.003" (0.0762 mm) |
| E4 | 4.79 | 2.16 | 0.04 | 0.23% | 4 | 14.6 | 4sx2p | |
| E6 | 3.97 | 2.98 | 0.03 | 0.17% | 6 | 9.7 | 4sx2p | |

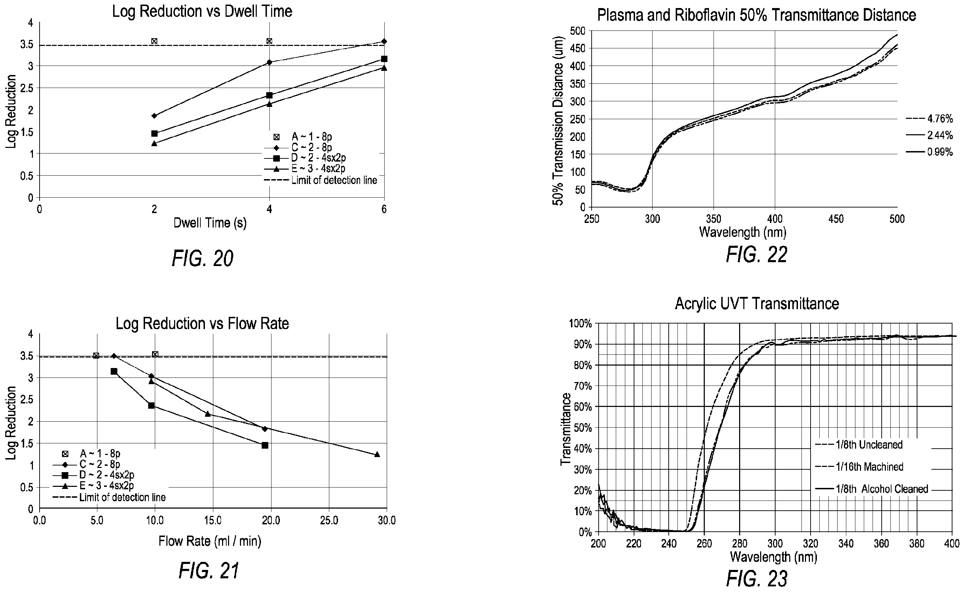

FIG. 20 plots log reduction vs dwell time. Pathogen reduction in flow cells C, D and E is linearly related to dwell time as expected. The 8p channel configuration results in the greatest pathogen reduction and the greatest rate of pathogen reduction (slope of pathogen reduction vs dwell time). It is not known why the 8p flow cell configuration is better than the 4px2s configuration.

Flow cell E results in less pathogen reduction than flow cell D likely because of the difference in channel thickness. The slope of pathogen reduction vs dwell time of flow cells D and E is the same as expected because both have the same channel configuration.

FIG. 21 plots log reduction vs flow rate. With respect to flow rate, flow cells C and E have similar performance with the best log reduction per flow rate. Flow cell E has thicker channels and a different cell configuration than flow cell C. Given that flow cell E has only a little less pathogen reduction for the same dwell time than flow cell D it may be that the same would hold true for flow cells with the 8p cell configuration. Therefore it may be that a flow cell with an 8p cell configuration and channel thickness of 0.003" (0.0762 mm) could outperform all flow cells tested in this study.

Hemolysis data is summarized in Table 3 above. Hemolysis for flow cells C, D and E is the same as the hold control or within 0.06% hemolysis of the hold control. Sample A2 shows no hemolysis but sample A4 has considerable hemolysis. This infers that the main cause of hemolysis is not shearing because sample A2 was at a higher flow rate than A4. The predicted cause of hemolysis is temperature because when flow cell A is removed from the fixture it was over 42°C. The effects of hemolysis due to over dosage cannot be ruled out because A4 received a higher dose than A2.

Flow cells are tested from thickest channel thickness to thinnest channel thickness. All flow cells are tested from low flow rate to high flow rate except for flow cell A. During flow cell A samples are taken from high flow rate to low flow rate to attempt to avoid clogging.

Two of the lamps do not turn on when the fixture is warming up for flow cell C. This error is fixed but it is unknown if the two lamps are operational for flow cell D.

Sample A6 is unobtainable because of flow cell clogging and breaks that leak air into the system. When the flow cell is removed from the illuminator it is 42°C. This high temperature may explain the flow cell clogging.

Flow cell B clogs early on. The only sample collected is B2. Sample B2 has many little bubbles in it and has visible hemolysis.

It is predicted that pathogen reduction is inversely exponentially related to channel thickness due to the transmittance of whole blood (light decays exponentially in a homogenous medium). This may be why the 0.003" (0.0762 mm) flow cell has less pathogen reduction than the 0.002" (0.0508 mm) flow cell for the same dwell time but the difference is small inferring that we are early on the exponential decay curve. Therefore, it is suggested to test 0.002" (0.0508 mm), 0.003" (0.0762 mm) and 0.005" (0.127 mm) channel flow cells. The thicker flow cells allow for higher flow rates and may be more manufacturable with injection molding. Theoretically there is an ideal channel thickness that optimizes flow rate and pathogen reduction.

This study shows the feasibility of flow through whole blood pathogen reduction. The data aligns with theory further validating the approach for whole blood flow through pathogen reduction. Pathogen reduction of the virus under test was within the acceptable pathogen reduction range. Further studies into thicker channels and other flow paths may be performed to optimize the system.

Example 2

This example provides information on the PhiX174 (pathogen) reduction observed in plasma with riboflavin at four different flow rates in four different prototype flow-through flow cells.

The goal of this study is to obtain insight into the correlation between flow rate, channel thickness and pathogen reduction. The flow cells in this study have a relatively thicker channel and different flow configurations.

The flow cell mounts in a fixture (e.g., flow cell holder) which maintains channel thickness by applying pressure from both the top and bottom. The fixture houses eight illumination lamps driven at 800 mA with four on top and four on bottom producing an irradiance of 4.5 mW/cm2. Product can be flowed through the flow-cell, which is mounted adjacent to the light source, and then samples are taken on the back end. Plasma combined with riboflavin and PhiX174 is the test medium (product).

The increased channel thickness results in decreased fluidic impedance requiring the use of a peristaltic pump to control flow rate instead of by gravity. The pump is calibrated prior to use to ensure flow rate accuracy.

1. PROTOCOL STEPS

1.1. Plasma Preparation (In biosaftey cabinet)

1.1.1. Obtain 1,100 mL of type matched plasma; record the BUI number of the units used on Appendix I.

1.1.2. Pool the plasma units into one bag.

1.1.3. Weigh the plasma, including the bag, record on the DCS.

1.1.4. Add 4 mL of PhiX174 inoculum to the plasma pool and record on the DCS.

1.1.5. Obtain one pouch of riboflavin for every unit of plasma used to create the pool.

1.1.6. Sterile connect each riboflavin pouch to the pool and drain the entire contents of the bag into the illumination set and document on the DCS. Purge all residual air into the empty riboflavin bags.

1.1.7. Aseptically remove three 2 mL samples from the plasma pool and transfer into three sample tubes labeled "PRE". These samples are used as hold controls for the study. Submit one PRE sample tube for viral titer and save the other two for later analysis.

1.2. Pump Preparation

1.2.1. Load the pump calibration tubing supplied with the disposables into the pump head.

1.2.2. Place an empty beaker onto a scale next to the outlet side of the pump.

1.2.3. Place the inlet side of the calibration tubing into deionized water and the outlet side of the pump into the beaker on the scale.

1.2.4. Prime the pump.

1.2.5. Select L/S 14 as the tubing type and set the flow rate to 20 mL/min.

1.2.6. Tare the scale with the beaker on it.

1.2.7. Press CAL and note the calibration volume that appears.

1.2.8. Press STOP/START and the pump will dispense the calibration volume into the beaker.

1.2.9. Once the pump has stopped, enter the weight of calibration volume that was dispensed into the pump using the UP/DOWN arrow keys.

1.2.10. Press SIZE to exit the calibration cycle.

1.3. Disposable Preparation (In biosaftey cabinet)

1.3.1. Obtain sterile prototype flow-through disposable, and a sterile flow-cell assembly.

1.3.2. Close all clamps on the set.

1.3.3. Connect the flow-cell to the disposable components.

1.3.4. Connect the reservoir bag with the plasma to the disposable components.

1.3.5. Connect a bag of saline to the disposable components.

1.4. Illumination Preparation

1.4.1. Label the first four sample bags with the corresponding sample name (Table 4). The fifth sample bag is intentionally not used and intended to be an extra in the event that an extra sample bag is required.

1.4.2. Mount the flow-cell and ensure the flow cell is properly loaded into the enclosure.

1.4.3. Open the clamps to allow flow from the saline bag to the waste bag. All other clamps should be closed.

1.4.4. Prime the flow-cell with saline with a flow rate equal to or less than the maximum sample flow rate.

1.4.5. Ensure all other clamps are closed.

1.4.6. Prepare the illumination setup.

1.4.7. Apply UV protective safety glasses to all personnel working in the area.

1.4.8. Ensure proper light barriers are in place to minimize UV light expose.

1.4.9. Turn on the UV lamps and cooling fan and allow 10 minutes for the lamp to warm up.

Table 4: Naming Convention

| Flow | Flow Rate (mL/min) | | | | | | Flow | Spacer Thickness |
| Cell | 5 | 10 | 20 | 40 | 80 | | Cell | (Thousands of an inch) |
|---|---|---|---|---|---|---|---|---|
| A | A5 | A10 | A20 | A40 | | | A | 3 |
| B | B5 | B10 | B20 | B40 | | | B | 5 |
| C | | C10 | C20 | C40 | C80 | | C | 12 |
| D | | | D20 | D40 | D80 | | D | 20 |

1.5. Sample Generation

1.5.1. Ensure the lamps are warmed up and have been running for 10 minutes.
1.5.2. Purge the system and take sample.

Table 5: Flow Rate Timing

| Flow Rate (mL/min) | Purge Time | | Sample Time | |
| | Minutes | Seconds | Minutes | Seconds |
|---|---|---|---|---|
| 5 | 8 | 480 | 3 | 180 |
| 10 | 4 | 240 | 1.5 | 90 |
| 20 | 2 | 120 | 0.75 | 45 |
| 40 | 1 | 60 | 0.375 | 22.5 |
| 80 | 0.5 | 30 | 0.1875 | 11.25 |

1.5.2.1. Ensure that the pump is programmed with the correct flow rate from Table 4.
1.5.2.2. Open all the clamps to allow flow from the plasma pool to the waste bag.
1.5.2.3. Turn on the pump and start the stop watch (approximately simultaneously).
1.5.2.4. Monitor fluid progress through the disposable.
1.5.2.5. After the purge time from Table 5 has elapsed (allowing at least 40 mL of plasma to flow into the waste bag) open the clamp to the corresponding sample bag then close the clamp to the waste bag.
1.5.2.6. After the sample time from Table 5 has elapsed (allowing at least 15 mL of plasma to flow into the sample bag) open the clamp to the waste bag then close the clamp to the sample bag. Open the clamp from the saline then close the clamp from the plasma bag.
1.5.3. Repeat from step 1.5.2 for each flow rate in Table 4.
1.5.4. Seal and remove all sample bags. Aseptically remove three 2 mL samples from each bag and transfer into three sample tubes labeled as indicated in Table 3.
1.5.5. Submit one sample tube for each sample for viral titer and save the other two samples for later analysis.
1.5.6. Repeat from step 1.3 for each flow cell. Use the same plasma pool for the entire study.
1.5.7. Aseptically remove three 2 mL samples from the plasma pool and transfer into three sample tubes labeled "HC". These samples will be used as hold controls for the study. Submit one HC sample tube for viral titer and save the other two for later analysis.

1.6. Sample Handling

1.6.1. Transfer samples into a labeled 2 mL cryovial.
1.6.2. Place the cryovials into an ultra-freezer (-80°C) for analysis at a later date.

Below are the pathogen reduction results. In Table 6, cells with bold text indicate a sample that is within the limit of detection and cells with an asterisk indicate a sample that has six or less PhiX174 colonies at a one log dilution and is outside the limit of detection. Cells with underlined text indicate a sample is technically outside the limit of detection but has PhiX174 colonies allowing for the inferred calculation of pathogen reduction. Each cell also has dwell time in seconds and predicted dose. The lower predicted dose value takes into account the transmissivity of the plasma and flow cell. Transmissivity measurements of the plasma and flow cells are graphed and shown in FIG. 22 and FIG. 23. The higher predicted dose assumes that all light incident on the flow cell is absorbed by the plasma.

With respect to FIG. 22, samples are prepared by mixing one unit of plasma with one 35 mL pouch of riboflavin and then diluting the samples with saline. The percentages above denote the concentration of plasma. Concentration and transmission distance are taken into account when the 50% transmission distance is calculated. The Beer-Lambert law is used to determine 50% transmission distance using the equations below.

$$T = (e^{-\varepsilon bc})$$

T = transmissivity
ε = absorbtivity/concentration
b = path length
c = concentration

Table 6: Summary of Results

| Gap Thickness (1/1000") | Flow Rate (ml/min) | | | | |
|---|---|---|---|---|---|
| | 5 | 10 | 20 | 40 | 80 |
| 3 (A) (0.0762 mm) | *12 sec* 5.9 - 20.3 J/ml | *5.8 sec* 3.0 -10.2 J/ml | 2.9 sec (~3.4 logs)* 1.5 - 5.1 J/ml | **1.5 sec (2.8 logs) * .7 -2.5 J/ml** | |
| 5 (B) (0.127 mm) | *19 sec* 14.9 -33.9 J/ml | *9.7 sec* 7.4 -16.9 J/ml | 4.9 sec (~3.6 logs)* 3.7 - 8.5 J/ml | **2.43 sec (2.6 logs)* 1.9 - 4.2 J/ml** | |
| 12 (C.) (0.305 mm) | | *23 sec* 30.4 -40.6 J/ml | *11 sec* 15.2 -20.3 J/ml | 5.8 sec (~3.9 logs) * 7.6 -10.1 J/ml | **2.9 sec (2.5 logs) * 3.8 - 5.1 J/ml** |
| 20 (D) (0.508 mm) | | | *19 sec* 30.5 -33.9 J/ml | *9.7 sec* 15.3 -16.9 J/ml | **4.7 sec (2.9 logs) * 7.6- 8.5 J/ml** |

Greater pathogen reduction than anticipated is achieved resulting in the majority of the data points being beyond the limit of detection. Therefore, no major conclusions about the correlation between channel thickness, flow rate and pathogen reduction are made. The data points that are within the method detection limits have a correlation between dwell time and pathogen reduction. The greater the dwell time the greater the pathogen reduction.

The 0.005" (0.127 mm) thick channel flow cell has opaque deposits on certain sections of the flow cell on the inside of the flow cell after illumination. This indicates that the plasma is overdosed possibly due to the flow stopping in certain regions of the flow cell. It is observed that the 0.005" (0.127 mm) thick flow cell has the most issues with priming due to bubbles in the system.

All flow cells achieve adequate pathogen reduction demonstrating the feasibility of flow through pathogen reduction of plasma and riboflavin. Much of the data is beyond the limit of detection limiting the conclusions that can be drawn from the data in terms of effects of channels thickness and flow rate on pathogen reduction.

It may be apparent to those skilled in the art that various modifications and variations can be made to the embodiments of the present invention described above without departing from the scope of the claims. Thus it should be understood that the invention is not be limited to the specific examples given, the embodiments described, or the embodiments shown in the figures. Rather, the invention is intended to cover modifications and variations within the scope of the appended claims.

While example embodiments and applications of the present invention have been illustrated and described, it is to be understood that the invention is not limited to the precise configuration, steps, and structures described above. Various modifications, changes, and variations apparent to those skilled in the art may be made in the arrangement, operation, and details of the methods and systems of the present invention disclosed herein without departing from the scope of the invention as defined by the appended claims.

**Claims**

1. A flow cell (200) for reducing one or more pathogens in a fluid, the flow cell (200) comprising:

   a plurality of channels (212), each of the plurality of channels (212) comprising:

   a depth (212A) of less than 0.150 mm;
   a first wall transparent to ultraviolet light;
   a second wall transparent to ultraviolet light;

   an inlet port (220), wherein fluid enters the flow cell (200) through the inlet port (220);
   at least one inlet manifold (216) in fluid communication with the inlet port (220) and the plurality of channels (212), wherein a cross section of the at least one inlet manifold (216) tapers from a proximal end (216A) of the at least one inlet manifold (216) to a distal end (216B) of the at least one inlet manifold (216);
   an outlet port (232) wherein fluid exits the flow cell (200) through the outlet port (232);
   at least one outlet manifold (224, 228) in fluid communication with the outlet port (232) and the plurality of channels, (212) wherein a cross section of the at least one outlet manifold (224, 228) tapers from a proximal end (224A, 228A) of the at least one outlet manifold (224, 228) to a distal end (224B, 228B) of the at least one outlet manifold (224A, 228A).

2. The flow cell (200) of claim 1, wherein the fluid comprises one or more of red blood cells, plasma, platelets, or combinations thereof.

3. The flow cell (200) of claim 1, wherein the fluid comprises packed red blood cells.

4. The flow cell (200) of any one of claims 1-3, wherein at least one of the plurality of channels (212) fluidly connects the proximal end (216A) of the inlet manifold (216) to the distal end (224B, 228B) of the outlet manifold (224, 228).

5. The flow cell (200) of any one of claims 1-4, wherein at least one of the plurality of channels (212) fluidly connects the distal end (216B) of the inlet manifold (216) to the proximal end (224A, 228A) of the outlet manifold (224, 228).

6. The flow cell (200) of any one of claims 1-5, wherein the flow cell (200) is constructed from two pieces (204, 208), and wherein a first piece (204) comprises: the at least one inlet manifold (216), the at least one outlet manifold (224, 228), and the first wall of each of the plurality of channels (212).

7. The flow cell (200) of claim 6, wherein a second piece (208) comprises: a plurality of walls (236) for creating the depth (212A) of each of the plurality of channels (212) and the second wall of each of the plurality of channels (212).

8. The flow cell (200) of claim 7, wherein the plurality of channels (212) each comprises a cross section, perpendicular to a length of the channel, that is substantially rectangular.

9. The flow cell of claim 7, wherein the first piece further comprises: the inlet port and the outlet port.

10. The flow cell (200) of claim 7, wherein a perimeter of the first piece (204) is attached to a perimeter of the second piece (208).

11. The flow cell of claim 7, wherein the first piece (204) and the second piece (208) are made from polymeric material.

12. The flow cell of claim 11, wherein the polymeric material comprises acrylic.

13. The flow cell (200) of any one of claims 1-12, wherein the flow cell (200) is disposable.

14. The flow cell (200) of any one of claims 1-13, further comprising tubing that fluidly connects the outlet port (232) to a bag for storing fluid that exits the flow cell (200) through the outlet port (232).

**15.** A system for reducing one or more pathogens in a fluid, the system comprising:

the flow cell of any one of the preceding claims; and
an illumination system, wherein the illumination system illuminates each of the plurality of flow channels from a plurality of sides.

**Patentansprüche**

**1.** Durchflusszelle (200) zum Reduzieren eines oder mehrerer Pathogene in einem Fluid, wobei die Durchflusszelle (200) Folgendes umfasst:

eine Vielzahl von Kanälen (212), wobei jeder der Vielzahl von Kanälen (212) Folgendes umfasst:

eine Tiefe (212A) von weniger als 0,150 mm,
eine erste Wand, die für ultraviolettes Licht durchlässig ist;
eine zweite Wand, die für ultraviolettes Licht durchlässig ist;

eine Einlassöffnung (220), wobei Fluid durch die Einlassöffnung (220) in die Durchflusszelle (200) eintritt;
mindestens einen Einlassverteiler (216) in Fluidverbindung mit der Einlassöffnung (220) und der Vielzahl von Kanälen (212), wobei sich ein Querschnitt des mindestens einen Einlassverteilers (216) von einem proximalen Ende (216A) des mindestens einen Einlassverteilers (216) zu einem distalen Ende (216B) des mindestens einen Einlassverteilers (216) hin verjüngt;
eine Auslassöffnung (232), wobei Fluid die Durchflusszelle (200) durch die Auslassöffnung (232) verlässt;
mindestens einen Auslassverteiler (224, 228) in Fluidverbindung mit der Auslassöffnung (232) und der Vielzahl von Kanälen (212), wobei ein Querschnitt des mindestens einen Auslassverteilers (224, 228) sich von einem proximalen Ende (224A, 228A) des mindestens einen Auslassverteilers (224, 228) zu einem distalen Ende (224B, 228B) des mindestens einen Auslassverteilers (224A, 228A) hin verjüngt.

**2.** Durchflusszelle (200) nach Anspruch 1, wobei das Fluid eines oder mehrere von roten Blutzellen, Plasma, Blutplättchen oder Kombinationen davon umfasst.

**3.** Durchflusszelle (200) nach Anspruch 1, wobei das Fluid konzentrierte Erythrozyten umfasst.

**4.** Durchflusszelle (200) nach einem der Ansprüche 1 bis 3, wobei mindestens einer der Vielzahl von Kanälen (212) das proximale Ende (216A) des Einlassverteilers (216) mit dem distalen Ende (224B, 228B) des Auslassverteilers (224, 228) fluidisch verbindet.

**5.** Durchflusszelle (200) nach einem der Ansprüche 1 bis 4, wobei mindestens einer der Vielzahl von Kanälen (212) das distale Ende (216B) des Einlassverteilers (216) mit dem proximalen Ende (224A, 228A) des Auslassverteilers (224, 228) fluidisch verbindet.

**6.** Durchflusszelle (200) nach einem der Ansprüche 1 bis 5, wobei die Durchflusszelle (200) aus zwei Teilen (204, 208) aufgebaut ist und wobei ein erstes Teil (204) Folgendes umfasst: den mindestens einen Einlassverteiler (216), den mindestens einen Auslassverteiler (224, 228) und die erste Wand von jedem der Vielzahl von Kanälen (212).

**7.** Durchflusszelle (200) nach Anspruch 6, wobei ein zweites Teil (208) Folgendes umfasst: eine Vielzahl von Wänden (236) zum Erzeugen der Tiefe (212A) von jedem der Vielzahl von Kanälen (212) und der zweiten Wand von jedem der Vielzahl von Kanälen (212).

**8.** Durchflusszelle (200) nach Anspruch 7, wobei die Vielzahl von Kanälen (212) jeweils einen Querschnitt senkrecht zu einer Länge des Kanals aufweist, der im Wesentlichen rechteckig ist.

**9.** Durchflusszelle nach Anspruch 7, wobei das erste Teil ferner Folgendes umfasst: die Einlassöffnung und die Auslassöffnung.

**10.** Durchflusszelle (200) nach Anspruch 7, wobei ein Umfang des ersten Teils (204) an einem Umfang des zweiten Teils (208) angebracht ist.

**11.** Durchflusszelle nach Anspruch 7, wobei das erste Teil (204) und das zweite Teil (208) aus Polymermaterial bestehen.

**12.** Durchflusszelle nach Anspruch 11, wobei das Polymermaterial Acryl umfasst.

**13.** Durchflusszelle (200) nach einem der Ansprüche 1 bis 12, wobei die Durchflusszelle (200) eine Einwegzelle ist.

**14.** Durchflusszelle (200) nach einem der Ansprüche 1 bis 13, ferner umfassend eine Schlauchleitung, die die Auslassöffnung (232) fluidisch mit einem Beutel zum Speichern eines Fluids verbindet, das die Durchflusszelle (200) durch die Auslassöffnung (232) verlässt.

**15.** System zur Reduzierung eines oder mehrerer Pathogene in einem Fluid, wobei das System Folgendes umfasst:

die Durchflusszelle nach einem der vorhergehenden Ansprüche; und
ein Beleuchtungssystem, wobei das Beleuchtungssystem jeden der Vielzahl von Durchflusskanälen von einer Vielzahl von Seiten beleuchtet.


**Revendications**

**1.** Cuve à circulation (200) pour réduire un ou plusieurs agents pathogènes dans un fluide, la cuve à circulation (200) comprenant :

une pluralité de canaux (212), chacun de la pluralité de canaux (212) comprenant :

une profondeur (212A) inférieure à 0,150 mm ;
une première paroi transparente à la lumière ultraviolette ;
une deuxième paroi transparente à la lumière ultraviolette ;

un orifice d'entrée (220), dans laquelle du fluide pénètre dans la cuve à circulation (200) par l'orifice d'entrée (220) ;
au moins un collecteur d'entrée (216) en communication fluidique avec l'orifice d'entrée (220) et la pluralité de canaux (212), dans lequel une section transversale de l'au moins un collecteur d'entrée (216) se rétrécit d'une extrémité proximale (216A) de l'au moins un collecteur d'entrée (216) à une extrémité distale (216B) de l'au moins un collecteur d'entrée (216) ;
un orifice de sortie (232), dans laquelle du fluide sort de la cuve à circulation (200) par l'orifice de sortie (232) ;
au moins un collecteur de sortie (224, 228) en communication fluidique avec l'orifice de sortie (232) et la pluralité de canaux (212), dans lequel une section transversale de l'au moins un collecteur de sortie (224, 228) se rétrécit d'une extrémité proximale (224A, 228A) de l'au moins un collecteur de sortie (224, 228) à une extrémité distale (224B, 228B) de l'au moins un collecteur de sortie (224A, 228A).

**2.** Cuve à circulation (200) selon la revendication 1, dans laquelle le fluide comprend un ou plusieurs parmi des globules rouges, du plasma, des plaquettes, ou des combinaisons de ceux-ci.

**3.** Cuve à circulation (200) selon la revendication 1, dans laquelle le fluide comprend un concentré de globules rouges.

**4.** Cuve à circulation (200) selon l'une quelconque des revendications 1 à 3, dans laquelle au moins l'un de la pluralité de canaux (212) relie fluidiquement l'extrémité proximale (216A) du collecteur d'entrée (216) à l'extrémité distale (224B, 228B) du collecteur de sortie (224, 228).

**5.** Cuve à circulation (200) selon l'une quelconque des revendications 1 à 4, dans laquelle au moins l'un de la pluralité de canaux (212) relie fluidiquement l'extrémité distale (216B) du collecteur d'entrée (216) à l'extrémité proximale (224A, 228A) du collecteur de sortie (224, 228).

**6.** Cuve à circulation (200) selon l'une quelconque des revendications 1 à 5, dans laquelle la cuve à circulation (200) est construite à partir de deux pièces (204, 208), et dans laquelle une première pièce (204) comprend : l'au moins un collecteur d'entrée (216), l'au moins un collecteur de sortie (224, 228), et la première paroi de chacun de la pluralité de canaux (212).

**7.** Cuve à circulation (200) selon la revendication 6, dans laquelle une deuxième pièce (208) comprend : une pluralité de parois (236) pour créer la profondeur (212A) de chacune de la pluralité de canaux (212) et la deuxième paroi de chacun de la pluralité de canaux (212).

**8.** Cuve à circulation (200) selon la revendication 7, dans laquelle la pluralité de canaux (212) comprennent chacun une section transversale, perpendiculaire à la longueur du canal, qui est substantiellement rectangulaire.

**9.** Cuve à circulation selon la revendication 7, dans laquelle la première pièce comprend en outre : l'orifice d'entrée et l'orifice de sortie.

**10.** Cuve à circulation (200) selon la revendication 7, dans laquelle un périmètre de la première pièce (204) est fixé à un périmètre de la deuxième pièce (208).

**11.** Cuve à circulation selon la revendication 7, dans laquelle la première pièce (204) et la deuxième pièce (208) sont fabriquées à partir d'un matériau polymère.

**12.** Cuve à circulation selon la revendication 11, dans laquelle le matériau polymère comprend un acrylique.

**13.** Cuve à circulation (200) selon l'une quelconque des revendications 1 à 12, dans laquelle la cuve à circulation (200) est jetable.

**14.** Cuve à circulation (200) selon l'une quelconque des revendications 1 à 13, comprenant en outre une tubulure qui relie fluidiquement l'orifice de sortie (232) à une poche pour stocker du fluide qui sort de la cuve à circulation (200) par l'orifice de sortie (232).

**15.** Système pour réduire un ou plusieurs agents pathogènes dans un fluide, le système comprenant :

la cuve à circulation de l'une quelconque des revendications précédentes ; et
un système d'illumination, dans lequel le système d'illumination illumine chacun de la pluralité de canaux d'écoulement à partir d'une pluralité de côtés.

FIG. 1B

FIG. 1A

FIG. 2

FIG. 3

FIG. 4

FIG. 5

FIG. 6

FIG. 7A

FIG. 7B

FIG. 7C

FIG. 8

FIG. 9

FIG. 10

FIG. 11B

FIG. 11A

FIG. 12A

FIG. 12B

FIG. 13

FIG. 14

FIG. 16

FIG. 15

FIG. 17

FIG. 18

FIG. 19

Log Reduction vs Dwell Time

Log Reduction

Dwell Time (s)

A ~ 1 - 8p
C ~ 2 - 8p
D ~ 2 - 4sx2p
E ~ 3 - 4sx2p
Limit of detection line

FIG. 20

Log Reduction vs Flow Rate

Log Reduction

Flow Rate (ml / min)

A ~ 1 - 8p
C ~ 2 - 8p
D ~ 2 - 4sx2p
E ~ 3 - 4sx2p
Limit of detection line

FIG. 21

Plasma and Riboflavin 50% Transmittance Distance

50% Transmission Distance (um)

Wavelength (nm)

4.76%
2.44%
0.99%

FIG. 22

Acrylic UVT Transmittance

Transmittance

Wavelength (nm)

1/8th Uncleaned
1/16th Machined
1/8th Alcohol Cleaned

FIG. 23

FIG. 24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009079383 A1